(19) **Europäisches Patentamt**

**European Patent Office**

**Office européen des brevets**

(11) **EP 0 610 247 B1**

(12) **EUROPÄISCHE PATENTSCHRIFT**

(45) Date of publication and mention
of the grant of the patent:
**02.11.2000 Bulletin 2000/44**

(21) Application number: **92920625.8**

(22) Date of filing: **22.09.1992**

(51) Int. Cl.⁷: $A61B\ 17/32$, $A61F\ 9/00$

(86) International application number:
**PCT/US92/07947**

(87) International publication number:
**WO 93/05718 (01.04.1993 Gazette 1993/09)**

(54) **INTRAOCULAR SURGICAL SCISSORS**

INTRAOKULARE, CHIRURGISCHE SCHEREN

CISEAUX CHIRURGICAUX INTRAOCULAIRES

(84) Designated Contracting States:
**AT CH DE ES FR GB IT LI NL SE**

(30) Priority: **23.09.1991 US 764518**

(43) Date of publication of application:
**17.08.1994 Bulletin 1994/33**

(73) Proprietor: **GRIESHABER & CO. AG**
**CH-8203 Schaffhausen (CH)**

(72) Inventors:
• **PACKO, Kirk, H.**
**Flossmoor, IL 60422 (US)**
• **LO, Thomas, Ying-Ching**
**Fremont, CA 94539 (US)**
• **TAO, Franklin**
**Fremont, CA 94555 (US)**
• **ESCORCIO, Tolentino**
**San Leandro, CA 94577 (US)**

(74) Representative: **Althoff, Gerhard**
**Althoff Patentanwaltsbüro**
**Lättenstrasse 6A**
**Postfach**
**8185 Winkel/Bülach (CH)**

(56) References cited:
| | |
|---|---|
| DE-A- 3 902 109 | SU-A- 835 436 |
| US-A- 3 809 093 | US-A- 3 832 776 |
| US-A- 3 899 829 | US-A- 4 428 748 |
| US-A- 4 672 965 | US-A- 4 838 853 |
| US-A- 4 877 026 | US-A- 4 911 161 |

• KLIN. MBL. AUGENHEILK., no.166, 1975 pages
557 - 558 SPITZNAS, M. KRIEGER, A. 'Vibration
als neuartiges Schneideprinzip für die
Augenchirurgie'

**Description**

The invention relates to an electromechanical drive system for a device for carrying out intraocular surgical cutting functions and to the device which is provided with two scissor elements arranged parallel to one another in a guide tube and in which one scissor element is linearly displaceable relative to the other scissor element.

**Background of the invention**

Cutting instruments of many designs in the form of scissors, forceps, knives or the like are generally known for use in intraocular surgery, in which the drive can be carried out, for example, manually or with pneumatic pressure means or with an electric motor drive. The drive systems which can be activated by the surgeon in different ways, for example by a foot pedal, and cooperate with the respective cutting instrument are also known.

An instrument for cutting the front lens capsule is known, for example, from US-A 4,911,161, which comprises a housing designed as a hand grip, a headpiece arranged at one end of the housing and a guide tube which is mounted therein and is designed to receive a rod, wherein the rod cooperates at the end arranged in the housing via a coupling element with a magnet body capable of being driven in an axially oscillating manner and is provided at the other end with a cutting head which is bent at an obtuse angle to the longitudinal axis of the rod and is provided with four conically tapering cutting faces and cutting edges in the direction of a point starting from the curved portion. An axially orientated movement of two scissor elements capable of moving relative to one another and designed as cutting blades at the front end is not proposed in this device.

Vibratory scissors which are designed for use in cataract surgery are also known from US-A 3,899,829 or the documentary publication, "Vibration als neuartiges Schneideprinzip für die Augenchirurgie" (Klin. Mbl. Augenheilkunde 166 of 1975; pages 557 to 558, M.Spitznas; A.Kreiger), which comprise a housing designed as a hand grip, a drive system arranged therein and two scissor blades which are mutually spaced on the face from the housing, are angled in design cooperate with one another in the front region via a pin and are angled downwards with respect to the longitudinal axis of the housing at a concave angle, one lower scissor blade being stationary and the other, superimposed scissor blade being displaced with an a.c. current frequency of about 50 Hz relative to the stationary scissor blade and being pivoted round the axis of the pin. The angled scissor blades of these vibratory scissors require a relatively great incision for introduction into the eye and are designed with appropriate stiffness for cutting tissue.

A cutting instrument is also known from US-A 4,877,026 which comprises a first housing portion designed as a cylindrical hand grip, a second housing portion which is mounted therein in a sealed manner with a first portion and is provided with a conically tapering portion, and a guide tube mounted coaxially in the second housing portion, wherein a first scissor element which cooperates at one end via a rod and coupling element with an electrical, pneumatic or hydraulic drive and provided at the front free end with a first cutting blade as well as a second scissor element arranged substantially stationarily at one end in the second housing and is provided at the front free end with a second cutting blade is arranged in the guide tube.

With known cutting instruments having two scissor elements capable of moving linearly and relative to one another, the tissue, in particular the intraocular tissue, is expelled during closure of the cutting blades owing to the movement of the point of intersection and is deformed such that irregular, substantially S-shaped cuts or cuts with projecting edges or steps can be formed in a disadvantageous manner. Furthermore, during the closing movement of the scissor elements, the tissue can be clamped at the tips thereof by the lateral pressure so a perfect cut of the intraocular tissue is not guaranteed.

An object of the present invention is to provide a drive system for a device for carrying out microsurgical cutting functions with which movements which can be selected, in particular, as a function of the oscillation frequency of the tissue, can be carried out.

A further object of the present invention is to provide a device which is designed as a hand grip, a driving motor arranged therein and scissor elements which are correspondingly designed and are displaceable relative to one another, by means of which cuts which are uniform for the surgical operation can be made in the tissue, in particular in intraocular tissue, with a relatively high inherent oscillation frequency.

The solution to the problems and advantages of the invention will emerge from the following description given in conjunction with the drawings and the claims.

**Statement of the invention**

The present invention essentially relates to an electromechanical drive system and a device with two scissor elements which are each provided at the front end with a shaped cutting blade for cutting tissue. For selection of several cutting functions and for carrying them out, the drive system is connected to an electric circuit via a controller.

With the drive system according to the invention, at least one cutting element is moved in a linear and also vibrating and oscillating manner relative to the other cutting element for opening and closing the scissor blades arranged thereon. It is particularly important that the frequency of vibration is higher than the natural resonance of the tissue and rotation of the tissue is therefore prevented and continuous, almost rectilinear cuts can consequently be made in the tissue.

With the device according to the invention, the smaller cutting blade is sunk into the tissue during the cutting process so the device designed as a hand grip with the two scissor elements arranged thereon can be controlled very easily and changes of direction which may be required during cutting are also possible.

Various moving functions can be carried out with the drive system in conjunction with the controller and the individual elements of the electric circuit. During the respective functions, the natural oscillations of the living tissue are exceeded so an extremely high cutting force can be produced at the cutting edges of the cutting blades and the tissue can therefore be cut without any edges or steps.

As already mentioned, the cutting process is preferably carried out at a frequency which is higher than the natural oscillation frequency of the living tissue. The decisive frequency of the tissue can be defined by the following formula

$$f = \sqrt{\frac{k}{m}}$$

wherein **f** represents the oscillation, **k** the stiffness and m the mass of the tissue. It can be seen that the stiffer the tissue and the lower the mass m, the higher the tissue oscillation **f**. Intraocular tissues are known to have a relatively low mass m and stiffness **k** and thus a relatively high oscillation frequency.

The device according to the invention with the electromechanical drive system the driving motor and the specially designed cutting blades arranged toward one another of the two mutually parallel scissor elements guarantee a readily controllable efficient cutting process. The scissor elements arranged detachably in the device by means of a special mounting also guarantee a cut image with an almost uniform cut profile in which projecting edges or steps are substantially avoided even during successive cuts. Additional aids for holding the tissue are not required owing to the particular design of the two cutting blades.

Further advantages and features of the drive system according to the invention and of the device, etc., will emerge from the following description given in conjunction with the drawings and the claims.

**Description of the individual figures**

**Figure 1** is an exploded sectional view of a device with an insertable intraocular cutting instrument;

**Figure 2** is an elevation of the first bushing for the cutting instrument according to Figure 1;

**Figure 3** is a section along line 3-3 in Figure 2 of the first bushing;

**Figure 4** is a sectional view along line 4-4 of the first bushing;

**Figure 5** is an elevation of a first scissor element for the cutting instrument according to Figure 1;

**Figure 6** is a profile cross section along line 6-6 in Figure 5 of the cutting blade of the first scissor element;

**Figure 7** is an elevation of a second scissor element for the cutting instrument according to Figure 1;

**Figure 8** is a profile cross section along line 8-8 in Figure 7 of the cutting blade of the second scissor element;

**Figure 9** is a perspective view of the guide tube for the two scissor elements of the cutting instrument according to Figure 1;

**Figure 10** is a perspective view of a second bushing for the cutting instrument according to Figure 1;

**Figure 11a** is an enlarged view of the front portion of the two scissor elements for the cutting instrument according to Figure 1 in a first position;

**Figure 11b** shows the front portion of the two scissor elements in a second position;

**Figure 11c** shows the front portion of the two scissor elements in a third position;

**Figure 12** is a schematic elevation of a controller for a drive system of the device according to Figure 1 cooperating with a driving motor;

**Figure 13** is a circuit diagram of the electric circuit for the drive system cooperating with the driving motor;

**Figure 14** is a graph of the voltage trend of the electric circuit according to Figure 13;

**Figure 15** is a section through a housing for the device according to Figure 1 with the driving motor removed;

**Figure 16** is a section along line 16-16 in Figure 15 of the headpiece for the device according to Figure 1;

**Figure 17** is a section of the headpiece shown in Figure 16 along line 17-17;

**Figure 18** is a section through the driving motor for the device according to Figure 1;

**Figure 19** is a section through the device according to Figure 1 with inserted cutting instrument and scissor elements shown in the full open position;

**Figure 19a** shows the front portion designated by a circle K in Figure 19 with the scissor elements shown in the open position and on an enlarged scale;

**Figure 20** shows the device according to Figure 19 with the scissor elements shown in the closed position; and

**Figure 20a** shows the front portion designated by a circle K' in Figure 20 with the scissor elements shown in the closed position and on an enlarged scale.

**Description of an embodiment**

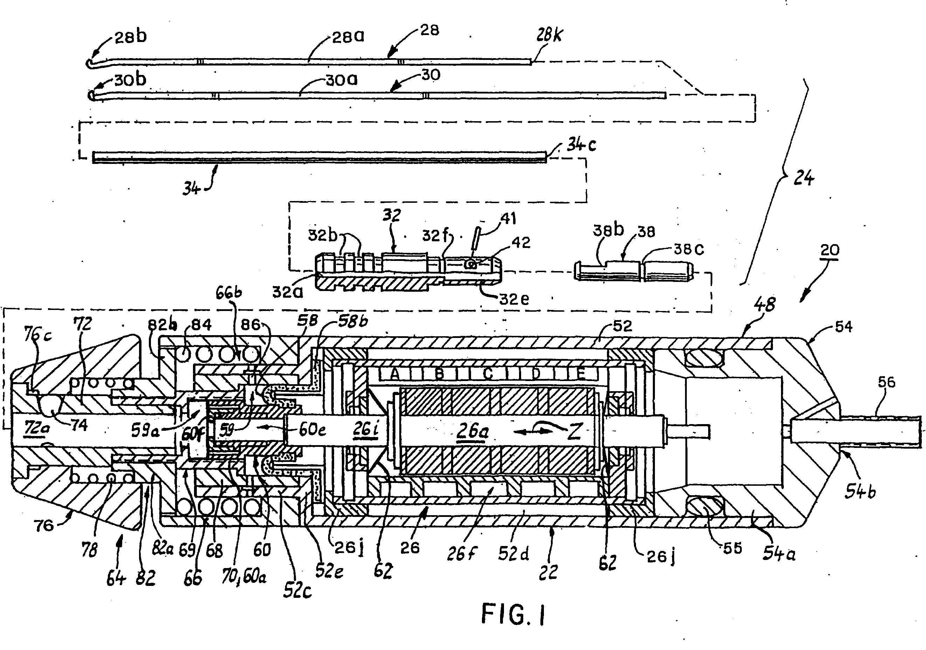

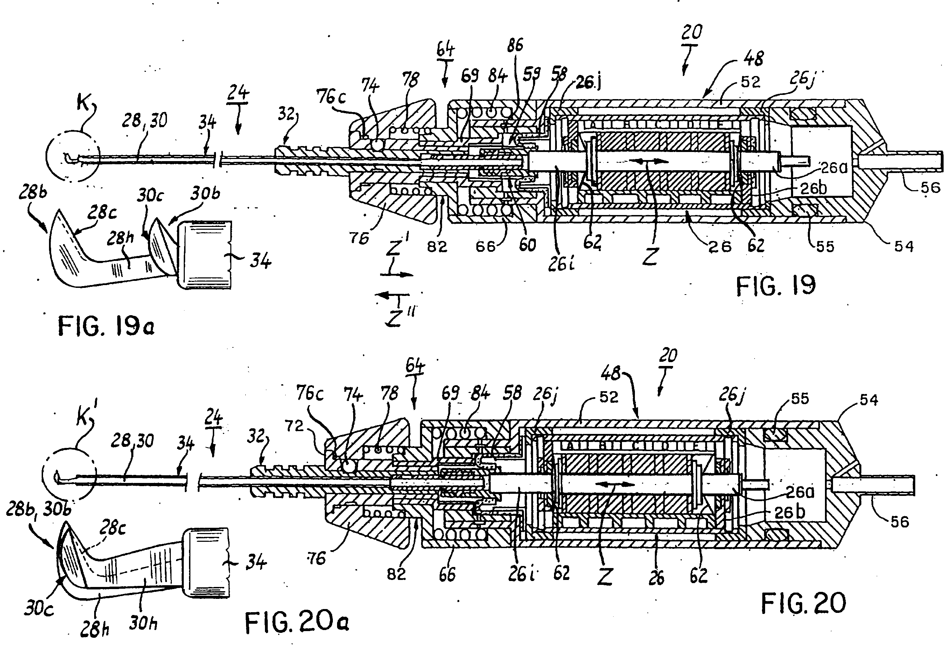

Figures 1 and 19 and 20 each show sections through a device 20 and a functional unit which cooperates therewith and will be described hereinafter as intraocular cutting instrument 24. The cutting instrument 24 which can be inserted into and removed from a mounting 64 is illustrated in an exploded view in Figure 1 and in the assembled state in Figures 19 and 20. The device 20 also comprises a housing 48 designed as a hand grip 22 and a driving motor 26 which is arranged therein and cooperates with the cutting instrument 24 detachably inserted into the mounting 26 as shown in Figures 19 and 20. The individual elements of the device 20, in particular the respective design of the housing 48, the driving motor 26 and the cutting instrument 24 as well as the mounting 64 for the cutting instrument 24 will be described in detail hereinafter.

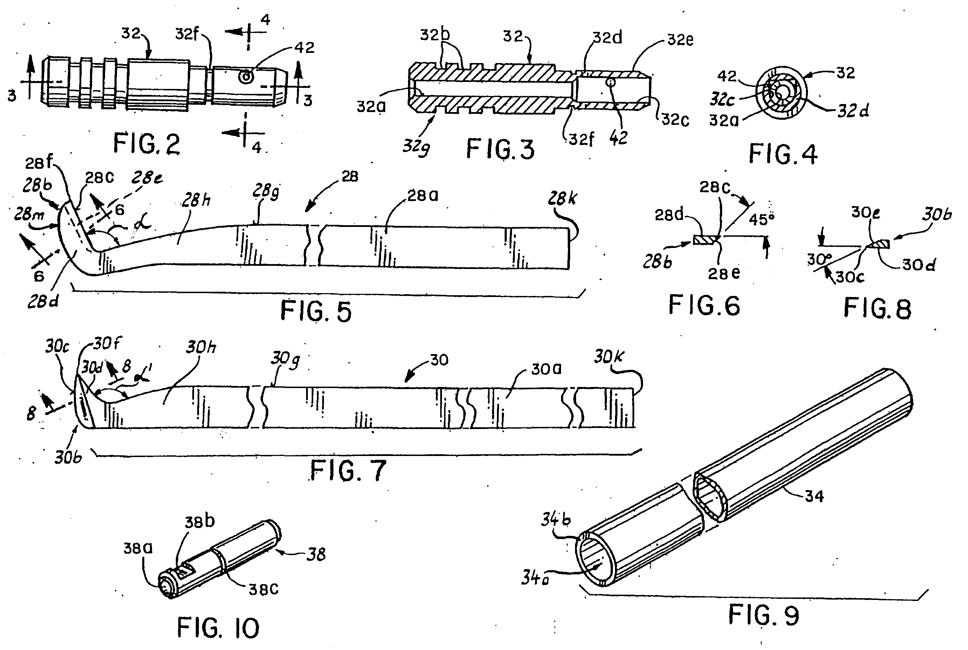

The cutting instrument 24 shown in the removed state and as an exploded view in Figure 1 comprises a first scissor element 28, a second scissor element 30, a guide tube 34 designed as a hollow needle to receive the two scissor elements 28 and 30 as well as a first bushing 32 and a second bushing 38. The first bushing 32 is provided with an axially orientated through-bore 32a in which the guide tube 34 can be inserted coaxially. As shown in Figures 19 and 20, the guide tube 34 is fastened to the first bushing 32 at the front face end by a welded joint (not shown). The guide tube 34 is designed to receive the two scissor elements 28 and 30 each provided with an elongate carrying arm 28a and 30a and with a respective cutting blade 28b and 30b at the front end. The first, shorter scissor element 28 with the rear end 28k (Figure 5) is rigidly connected to the end 34c of the guide tube 34 by a welded joint to avoid relative movement with respect to the second, longer scissor element 30. The parts 28,32 and 34 form a first constructional unit and the parts 30 and 38 also rigidly connected to one another by a welded joint form a second constructional unit. The design of the individual parts 28,30,32,34 and 38 is shown in Figures 2 to 10.

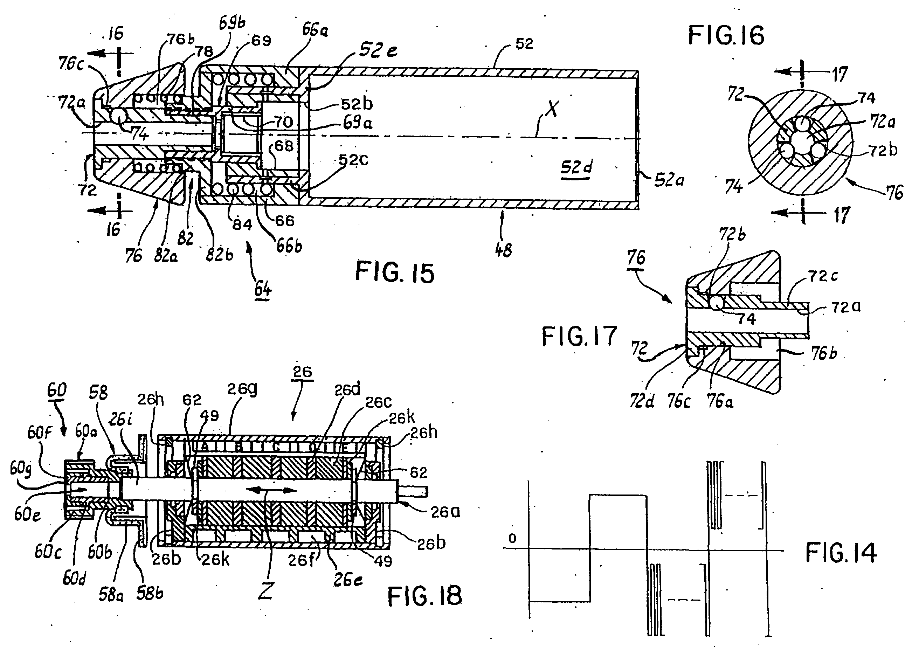

The device 20 shown in section in Figure 1 comprises the housing 48, the driving motor 26 which is arranged therein and is provided with a shaft 26a, the mounting 64 which is arranged on the front end of the housing 48 and forms a snap connection with the cutting instrument 24 as well as a closure member 54 arranged at the rear end of the housing 48. The individual elements of the mounting 64 and the individual elements cooperating with the driving motor 26, as well as the driving motor 26 will be described in detail hereinafter in conjunction with Figures 15 to 18. The closure member 54 designed substantially as a cap is arranged with a cylindrical portion 54a and a seal 55 (O-ring) arranged thereon in the interior 52d of the housing 48 designed as a cylindrical body 52. An electric cable 56 which is sealed by silicon, epoxide or the like and is connected in a manner not shown to the driving motor 26 arranged coaxially in the cylindrical body 52 is fastened on the back 54b of the closure member 54. As shown in Figure 1, the driving motor 26 is secured against axial displacement by two rings 26j which are axially spaced from one another in the interior 52d of the housing 48 and are correspondingly supported.

Figures 2 to 10 are respective enlarged views of the individual parts 28,30,32,34 and 38 of the intraocular cutting instrument, which will be described hereinafter.

The first bushing 32 shown in elevation in Figure 2 and in section in Figure 3 is provided with the axially orientated first through-bore 32a and with a second through-bore 32c connected thereto. Axially spaced furrows or grooves 32b are arranged on the outer periphery of the first, front portion 32d to improve handling. The rear, second portion 32d which is offset from the front, first portion is provided with a further annular groove 32f on the external diameter 32e. A bore 42 which is orientated transversely to the longitudinal direction of the bushing 32 and is arranged eccentrically with respect to the centre axis (not shown) is also arranged on the rear portion 32d. The bore 42 is provided for insertion of a pin 41 (Figure 1) by means of which the second bushing 38 which can be inserted into the second through-bore 32c in the assembled state cooperates with the first bushing 32. Figure 4 is a section along line 4-4 in Figure 2 of the first bushing 32, and shows the rear portion 32d provided with the second through-bore 32c, the first through-bore 32a as well as the bore 42 arranged transversely to the longitudinal direction of the first bushing 32.

Figure 5 is an enlarged view of the first scissor element 28 showing a portion 28h shaped at an inclination to the upper side 28g on the front end of the elongate carrying arm 28a and the cutting blade 28b which is inclined forwards from the back 28k. The cutting blade 28b has a flat or plane lateral wall 28d which is provided as a bevelled cutting edge 28c on one side and with a substantially curved cutting edge 28b on the opposite side. The cutting edge 28c orientated in the direction of a cutting tip 28f starting from the inclined portion 28h of the carrying arm 28a can also be concave, preferably slightly concave, in a manner not shown in detail. The cutting blade 28b is arranged and shaped on the inclined portion 28h of the carrying arm 28a such that the cutting edge 28c is arranged at an obtuse angle, for example at an angle $\alpha$, to the inclined portion 28h of the carrying arm 28a.

Figure 6 is a profile cross section of the cutting blade 28b shaped on the portion 28h of the carrying arm 28a along the line 6-6 shown in Figure 5, and shows the plane lateral wall 28d as well as the cutting face 28e of the cutting edge 28c ground to an angle of about 45°.

Figure 7 is an enlarged view of the second scissor element 30 showing a portion 30h inclined to the upper side 30g at the front end of the elongate carrying arm 30a and the cutting blade 30b inclined forwards from the back 30k. The cutting blade 30b has a flat or plane lateral wall 30d which is designed as a bevelled cutting edge 30c on one side. The cutting edge 30c which is orientated in the direction of a cutting tip 30f is preferably slightly convex or curved in design. The cutting blade 30b is arranged and shaped on the inclined portion 30h of the carrying arm 30a such that the lateral wall 30d is arranged, for example, at an obtuse angle at an angle $\alpha'$ to the inclined portion 30h of the carrying arm 30a.

Figure 8 is a profile cross section through the cutting blade 30b along line 8-8 in Figure 7, showing the plane lateral wall 30d and the cutting face 30e with the cutting edge 30c ground to an angle of about 30°.

Figure 9 is a perspective view of the guide tube 34 provided with a through-bore 34a orientated axially from the front edge 34b. The guide tube 34 is designed as a hollow needle for receiving the two scissor elements 28 and 30 arranged parallel to one another. In the assembled state, the guide tube 34 with the two scissor elements 28 and 30 is arranged coaxially in the through-bore 32a in the bushing 32, as shown in figures 19 and 20. The guide tube 34 is produced, for example, from an extremely small stainless steel tube having a gauge of about 0.91 mm (20 gauge).

Figure 10 is a perspective view of a second bushing 38 penetrated by an axially orientated through-bore 38a. A notch 38b orientated transversely to the longitudinal direction and an outer annular groove 38c axially spaced therefrom is provided at the front end on the outer periphery of the second bushing 38. The through-bore 38a serves to receive the second scissor element 30 which cooperates with the second bushing 38 in a manner not illustrated, for example by soldering or the like. The annular groove 38c arranged on the outer periphery is designed for a snap connection between the second bushing 38 and a coupling element 60 (Figures 19 and 20). The movements of the driving motor 26 are transmitted via the coupling element 60 onto the second bushing 38 or onto the second scissor element 30 cooperating therewith.

In the assembled state of the intraocular cutting instrument 24, the guide tube 34 is arranged in the first bushing 38 with the two scissor elements 28 and 30 arranged therein and is rigidly connected thereto (Figures 19 and 20). The second bushing 38 cooperating with the axially reciprocating second scissor element 30 is arranged with one end in the bore 32c in the first bushing 32 such that the pin 41 insertable into the bore 42 thereof (Figure 1) engages in the notch 38b in the second bushing 38. The axially orientated relative movement of the second bushing 38 with the second scissor element 30 with respect to the first bushing 32 and the first scissor element 28 cooperating therewith is limited according to the axial length of the notch 38b arranged on the second bushing 38.

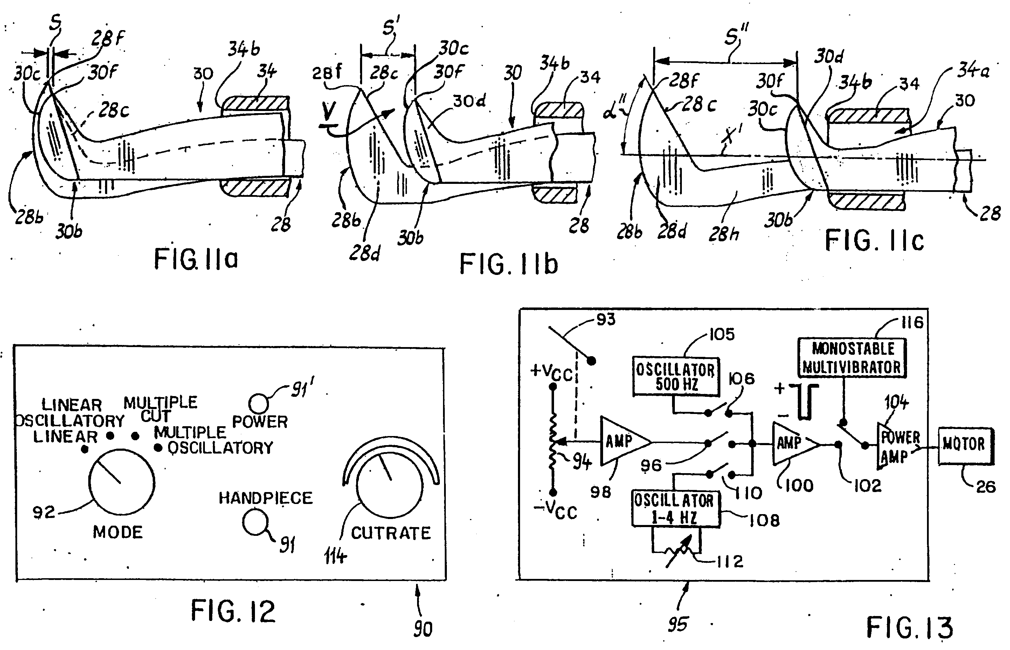

The functional procedures of the two scissor elements 28 and 30 of the cutting instrument 24 (Figure 1) are described in detail hereinafter in conjunction with Figures 11a,11b and 11c.

Figure 11a shows the two scissor elements 28 and 30 axially penetrating the guide tube 34 and arranged in parallel in the closed position. In the closed position, the second scissor element 30 is axially displaced relative to the front edge 34b of the guide tube 34 such that the cutting blade 30b of the second scissor element 30 is arranged to correspond to the cutting blade 28b of the first scissor element 28. The tips 28f and 30f of the two cutting blades 30b and 28b are arranged at a relatively short distance S of about 0.038 mm (.0015") from one another in the closed position. In the closed position, the cutting blade 30b of the second scissor element 30, as shown in Figure 11a, is pushed in an intersecting manner over the flat lateral wall 28d of the cutting blade 28b of the first scissor element 28. In this position, in which the cutting edges 30c and 28c of the two superimposed cutting blades 30b and 28b are mutually spaced (Figure 11a), the intraocular cutting instrument 24 can be brought into the respective cutting position (not shown) for the incision into the tissue, without unintentional and undesirable cutting of the adjacent tissue.

Figure 11b shows the two cutting blades 28b and 30b of the scissor elements 28 and 30 for the incision into the tissue in the partially open position. In this position, the two tips 28f and 30f of the cutting blades 28b and 30b are arranged at a distance S' of about 0.48 mm (.019") from one another. The two cutting edges 28c and 30c are arranged with a relatively small opening angle from one another by a substantially V-shaped gap V. The gap V between the two mutually facing cutting edges 28c and 30c substantially prevents undesirable expulsion of the tissue (not shown) which is to be cut and is in the gathered or puckered state during the cutting process. The convexly curved cutting edge 30c of the second scissor element 30 which is displaceable relative to the cutting edge 28c of the stationary first scissor element 28 also causes the tissue to be held between the two cutting edges 28c and 30c during the cutting process.

Figure 11c shows the two scissor elements 28 and 30 arranged in the through-bore 34a of the guide tube 34 in the fully open position in which the two tips 28f and 30f of the cutting blades 28b and 30b are arranged at a distance S'' of about 1.3 mm (0.0510'') from one another. As shown in Figure 11c, the first scissor element 28 is arranged in the guide tube 34 such that the cutting blade 28b shaped on the portion 28h is inclined forwards relative to the front edge 34b of the guide tube 34. The cutting edge 28c is arranged at an acute angle $\alpha''$ of about 60° to the theoretical longitudinal axis X' of the guide tube 34.

Figure 12 is a schematic view of a controller 90 for the device 20 which has a first adjusting button 91 (HAND PIECE), a second adjusting button 91' (POWER), a third adjusting button 92 (MODE) for the functions which are selectively adjustable by the surgeon and a control button 114 (CUT RATE) for setting the adjusted functions.

The controller 90 cooperates, in a manner not illustrated, with an electric circuit 95 shown as a circuit diagram in Figure 13, and the electric circuit 95 with the driving motor 26. The circuit 95 which can be activated by operating a foot pedal 93 comprises a potentiometer 94, several switches 96, 106, 110 and 102, several amplifiers 98, 100 and 104, a first oscillator 105 and a second oscillator 108 with associated control element 112 and a multivibrator 116 connected to the switch 102. The driving motor 26 of the device 20 can be activated by the above-mentioned elements. With the control device 90, the surgeon can select, for example, between four functions by corresponding actuation of the adjusting button 92, the individual functions and the advantages attainable therewith being described hereinafter.

The first function which can be adjusted with the adjusting button 92 of the controller 90 via the electric circuit 95 (Figure 13) produces a so-called linear mode (LINEAR MODE) in which an electric current is applied to the field winding 26f of the motor 26 and therefore axially displaces the shaft 26a. Owing to the axial displacement, the second scissor element 30 is moved relative to the first scissor element 28. The maximum displacement of the second scissor element 30 with respect to the first scissor element 28 is about 1.3 mm to 1.8 mm (.050'' to .070''), the control of the linear movement for the second scissor element 30 being achieved by operation of the foot pedal 93 cooperating with the potentiometer 94. A selected electrical d.c. voltage, for example, in the form of a d.c. signal is supplied during actuation of the foot pedal 93 via the first amplifier 98 to the closed first switch 96, the second amplifier 100, the closed double switch 102 and from the third amplifier 104 to the driving motor 26 (Figure 13).

With the first function (LINEAR MODE), the surgeon can accordingly control the linear movement for carrying out the individual cuts and therefore the relative movement of the second scissor element 30 with respect to the first scissor element 28 and bring about opening and closure thereof as a function of the distance (Figure 11b) required between the two scissor blades 30b and 28b.

The second function which can be adjusted with the adjusting button 92 of the controller 90 is a so-called linear oscillation movement (LINEAR OSCILLATORY) during which the control of the above-mentioned linear movement of the second scissor element 30 is achieved by actuating the foot pedal 93 cooperating with the potentiometer 94. The electrical direct current in the form of the corresponding d.c. signal is supplied by the electric circuit 95 via the first amplifier 98, the closed first switch 96 and via the closed second switch 106 to the first oscillator 105. The d.c. signal is supplied from the subsequent second amplifier 100 and a high-frequency oscillation signal from the first oscillator 105 via the double switch 102 and the third amplifier 104 to the driving motor 26 (Figure 13).

In conjunction with the second function, the scissor blade 30b of the second scissor element 30 can be positioned in any position with respect to the scissor blade 28b of the first scissor element 28 and can simultaneously be set into vibration by loading, brought about by the oscillator 105, of the driving motor 26 with a frequency of 500 Hz and relatively slight displacement between 0.0254 mm to 0.076 mm (.001'' to .003''). The relatively slight displacement of the scissor blade 30b cooperating with the shaft 26a also guarantees an optimum cutting process with different types of tissue. This also guarantees that the scissor blade 30b penetrates the tissue to be cut with a minimum of rotation or deviation.

The above-described second function (LINEAR OSCILLATORY) with the high-frequency vibrating movement in combination with the scissor blades 30b and 28b which are located in the partially open position has significant advantages in conjunction with the above-mentioned vibrating movement with respect to the point of intersection of the tissue to be cut. While allowing for these conditions, the tissue is puckered by slight lateral pressure against it and supplied to the point of intersection of the two open scissor blades 30b, 28b in a manner not illustrated. The surgeon can move the scissor blades 30b and 28b forward in the open position (Figure 11b) in order to cut the tissue along a smooth face with slight or insignificant deformation of the tissue. It is also possible to change the cutting direction without the tips 28f and 30f of the two scissor blades 30b and 28b sinking in the tissue.

The third function which can be adjusted with the adjusting button 92 of the controller 90 produces a so-called multiple cut (MULTIPLE CUT) during which the electric d.c. current in the form of the corresponding d.c. signal is supplied via the first amplifier 98 during operation of the foot pedal 93 to the closed first switch 96 and via the closed third switch 110 to the second oscillator 108 cooperating with the control element 112. The frequency of the second oscillator 108 which is between 1 and 4 Hz or between 60 and 240 oscillations per minute is variably adjustable via the control element 112 cooperating with the control button 114 of the controller 90 (Figure 12). The selected and adjusted output frequency (OUTPUT) is supplied to the driving motor 26 in the form of a corresponding d.c. signal via the double switch 102 and the third amplifier 104.

During the above-described third function (MULTIPLE CUT), the surgeon can move the second scissor element 30 to and fro relative to the first scissor element 28 at the frequency which is variable adjusted between 1 and 4 Hz by operating the foot pedal 93 (Figure 13). The particular design and arrangement of the mutually facing cutting edges 30c and 28c of the two scissor elements 30 and 28 in conjunction with the above-described driving functions allow improved cutting of tissue, in particular highly sensitive eye tissue. The gap V which widens substantially in the form of a V between the two cutting edges 28c and 30c (Figure 11b) allows the tissue to be cut to gather with reduced lateral pressure on the tissue during the forward movement of the device 20.

The fourth function which can be adjusted with the adjusting button 92 of the controller 90 produces a so-called multiple oscillatory movement (MULTIPLE OSCILLATORY) during which the two switches 106 and 110 of the electric circuit 95 are closed and the two oscillators 105 and 108 are connected via the second amplifier 100, the double switch 102 and the third amplifier 104 to the driving motor 26.

During the fourth function (MULTIPLE OSCILLATORY), the electric direct current is supplied in the form of the d.c. signal via the amplifier 98 and via the switches 96, 106, 110 to the first oscillator 105 and to the second oscillator 108. During this special function, the signals of the two oscillators 105,108 are connected to one another and supplied to the driving motor 26 in such a way that the two scissor elements 30, 28 are opened and closed with a frequency of 1 to 4 Hz and simultaneously with high-frequency oscillation or vibration.

When the foot pedal 93 is operated and the potentiometer 94 accordingly activated, the double switch 102 of the electric circuit 95 connected to a multivibrator 116 (MONOSTABLE MULTIVIBRATOR) is closed. When the foot pedal 93 is released, the double switch 102 is activated and a short negative signal supplied via the third amplifier 104 to the driving motor 26 by the multivibrator 116. The negative signal causes the second scissor element 30 to be moved firstly into the open position (Figure 11c) and then, owing to the positive d.c. signal, into the closed position (Figure 11a) on completion of the surgical operation.

Figure 14 shows the electrical voltage trend in the form of a graph in which the first two so-called square waves represent the signal produced during the function (MULTIPLE CUT) selected by the adjusting button 92 of the controller 90 (Figure 12) and supplied from the second oscillator 108 via the two amplifiers 100,104 to the driving motor 26 in amplified form.

During the function (MULTIPLE OSCILLATORY) selected with the adjusting button 92, the signal produced by the first oscillator 105 is superimposed by the signal of the second oscillator 108 so the square waves of the second oscillator 108 have a frequency of 2 Hz as shown in Figure 14, while the first oscillator 105 produces a frequency, superimposed by the signal of the second oscillator 108, of 500 Hz. The moving scissor element 30 can therefore be loaded with constant vibration in various positions simultaneously during opening and closure during the reciprocating movement.

Figure 15 is a section through the cylindrical body 52 designed as a housing 48 for the device 20 with the mounting 64 arranged thereon, the housing as well as the mounting 64 having a common longitudinal axis X. The cylindrical body 52 is provided at the front end with a portion 52c offset on a wall 52e for receiving the mounting 64 and at the rear end with an orifice 52a. The driving motor 26 can be inserted coaxially into the interior 52d and can be removed again through the orifice 52a (Figure 18). A bushing 68 designed to receive a hollow cylindrical bearing bush 69 is arranged in the cylindrical portion 52c of the cylindrical body 52 provided with an offset passage 52b. The bearing bush 69 has a first portion 69a mounted coaxially in the first bushing 68 and an axially orientated, second portion 69b which is offset thereon. The second portion 69b of the bearing bush 69 is mounted on a bushing 72 arranged in a headpiece 76 of the mounting 64. The first portion 69a of the bearing bush 69 has at least one, but preferably several, peripherally distributed internal guide grooves 70. In the assembled state, the guide grooves 70 engage (Figure 1) in an interlocking and frictional manner with guide webs 60a correspondingly distributed on the coupling element 60 of the driving motor 26 (Figure 18).

The mounting 64 arranged on the housing 48 comprises the headpiece 76, the bushing 72 mounted coaxially therein, an insert 66 and a disc-shaped supporting member 82. The bushing 72 provided with a through-bore 72a is mounted coaxially in a first bore 76a (Figure 17) of the headpiece 76 and designed to receive the second portion 69b of the bearing bush 69 on the rear offset portion 72c. The headpiece 76 is also provided with a recess 76b which is offset with respect to the first bore 76a and serves to receive a first compression spring 78. The headpiece 76 is mounted with the recess 76b on an offset portion 82a of the supporting member 82. The insert 66 is mounted with a shaped offset 66a on the cylindrical portion 52c of the housing 48 and is provided with a cylindrical recess 66b for receiving a second compression spring 84. The second compression spring 84 is supported by one end on the interior of the insert 66 and by the other end on a flange part 82b of the supporting member 82 (Figure 15).

Figure 16 is a section through the headpiece 76 along line 16-16 in Figure 15, and Figure 17 is a section along line 17-17 in Figure 16. The bushing 72 is arranged coaxially in the headpiece 76. The bushing 72 is provided, for example, with three peripherally distributed recesses 72b in which a ball 74 projecting partially into the through-bore 72a is arranged in each case.

Figure 17 also shows an annular groove 76c arranged at the front end of the headpiece 76 and the bushing 72 arranged with a flange 72d shaped on the front end in a recess (not shown) in the headpiece 76. Figures 16 and 17 also show the recesses 72b which taper conically inwards in the bushing 72 for the peripherally distributed balls 74 which are prevented from falling out by the conical shaping.

In the assembled state (Figures 19 and 20) the cutting instrument 24 comprising the two scissor elements 28 and 30 and the two bushings 32 and 38 and the guide tube 34 designed as a hollow needle is held by the balls 74 engaging in the annular groove 32f in the first bushing 32 (Figures 2,3). For removal or insertion of the cutting instrument 24, the headpiece 76 of the mounting 64 is displaced manually in the direction of the arrow Z' against the restoring force of the first compression spring 78 so the annular groove 76c of the headpiece 76 comes into engagement with the balls 74 which are moved radially outwards and disengage from the second bushing 32. After removal or insertion of the cutting instrument 24, the headpiece 76 is pushed back in the direction of the arrow Z'' by the restoring force of the compression spring 78.

Figure 18 is a section through the driving motor 26 which will be described in detail hereinafter. The driving motor 26 comprises the shaft 26a, two flanges 26b axially mutually spaced thereon and several magnet parts 26c which are arranged between the two flanges 26b and are separated from one another by intermediate rings 26d. The magnet part 26c and intermediate rings 26d are produced from a material of identical polarity. A respective disc 26k and a substantially C-shaped ring 49 is arranged at the two outer ends of the magnet parts 26c in each case. The intermediate rings 26d and magnet parts 26c arranged between the discs 26k are secured against axial displacement on the shaft 26a by the two rings 49. An axially resiliently acting disc 62 is arranged at each end between the two flanges 26b penetrated by the shaft 26a in each case and the associated rings 49. The magnet parts 26c which are axially spaced by the intermediate rings 26d are surrounded by a coil member 26e comprising several subdivisions 26f arranged axially in series for receiving individual coils A,B,C,D and E. The number of electrically connected coils A,B,C,D and E exceeds the number of magnet parts 26c by one coil. The shaft 26a with the magnet parts 26c arranged thereon and the coil member 26e resting on the flanges 26b are arranged coaxially in a cylindrical casing 26g. The coil member 26e is secured against axial displacement in the casing 26g by snap rings 26h penetrating in correspondingly provided annular grooves.

The coupling element designated in its entirety by 60 is arranged on the front end 26i of the shaft 26a. The coupling element 60 has a first cylindrical bearing body 60b with a bearing bush 60c shaped thereon in a radially externally offset manner. On the outer periphery of the bearing bush 60c there are arranged the peripherally distributed and axially orientated webs 60a which engage with the guide grooves 70 of the bearing bush 69 in the assembled state (Figure 1). A guide sleeve 60d is also provided which is arranged with an undesignated end in the bearing body 60b and is designed to receive a bearing ring 60f at the other end. The bearing ring 60f which is C-shaped in the profile cross section is provided with a radially inwardly orientated annular offset 60g which catches in the annular groove 38c of the second bushing 38 and therefore substantially cooperates with the cutting instrument 24 (Figures 19 and 20). The bushing 60d as well as the bearing ring 60f are penetrated by an axially orientated common bore 60e. The shaft 26a is arranged with one end piece 26i in an undesignated, offset through-bore in the bearing body 60b connected to the bore 60e (Figure 18).

As also shown in Figure 18, an undesignated outer annular groove which is designed to receive and hold a protective element 58 is provided on the outer periphery of the bearing body 60b. The protective element 58 has a radially outwardly orientated flange 58b and an annular part 58a which is shaped thereon and is substantially C-shaped in the profile cross section. The protective element 58 produced from elastically deformable material is held in an interlocking manner on the bearing body 60b by the annular part 58a. In the assembled state, the protective element 58 is clamped by the flange 58b in a manner not illustrated between the associated ring 26j and the wall 52e of the housing 48 (Figure 1). The driving motor 26 is protected from the admission of moisture by the protective element 58. With appropriate activation of the driving motor 26, the shaft 26a and the coupling element 60 connected thereto with the protective element 58 are moved to and fro in the direction of the double arrow Z.

The impacts possibly occurring in the end position during the reciprocating movement can be compensated by the two spring elements 62 arranged on the shaft 26a.

The above-described driving motor 26 is a so-called voltage transformer or linear servomotor with relatively low intensity and low resistance and special magnetic parts 26c by means of which at least one scissor element can be set into oscillation at a frequency of 1 to 1000 Hz. The magnetic parts 26c are preferably produced from neodymium iron boron (NdFeB) which guarantee adequate magnetic power in each case even after repeated steam sterilisation at relatively high temperature.

The shaft 26a of the driving motor 26 which is reciprocated in the direction of the arrow Z and the protective element 58 connected thereto via the coupling element 60 can produce a suction (vacuum) or a pressure surge during the surgical operation and transmit it via the guide tube 34 to the eye. To avoid this possibility, at least one suction orifice 86 is provided in the portion 52c of the cylindrical body 52 and in the bushing 68. Two diametrically opposed suction orifices 86 which radially penetrate the wall of the portion 52c and the wall of the bushing 68 and through which the two chambers 59 and 66b are connected to one another, as shown in figure 1, are preferably provided. Several peripherally distributed outlet orifices 86 are preferably provided.

Figure 19 is a section through the completely assembled device 20 showing the cutting instrument 24 which is inserted into the mounting 64 at the front end, the housing 48 provided with the closure member 54 at the rear end and the driving motor 26 which is arranged therein and is secured against axial displacement by the rings 26j. The shaft 26a cooperates with the coupling element 60 by means of the end piece 26i. In this position, an annular gap 59 is provided between the bearing bush 69 (Figure 1). The gap 59 corresponds substantially to the axially orientated relative movement of the second scissor element 30 with respect to the stationary first scissor element 28.

Figure 19a shows the front portion designated by a circle K in Figure 19 of the two scissor elements 28 and 30 arranged in the guide tube 34 in the fully open position. In this position, the two scissor blades 28b and 30b with the mutually facing cutting edges 28c and 30c arranged thereon are mutually spaced.

Figure 20 shows the device 20 with the essential functional elements as already described hereinbefore in conjunction with Figure 19. In contrast to Figure 19, the shaft 26a in the position according to Figure 20 is axially displaced by the corresponding activation of the driving motor 26 such that the curved portion of the protective element 58 which is correspondingly displaced in the gap 59 rests on the flange part, not shown, of the bearing bush 69. In this position, the scissor blades 28b and 30b of the two scissor elements 28,30 are arranged in the closed position relative to one another.

Figure 20a shows the front portion, designated by a circle K' in Figure 20, of the two scissor elements 28 and 29 arranged in the guide tube 34 in the closed position. In this position, the two scissor blades 28b and 30b with the cutting edges 28c and 30c arranged thereon are arranged in a superimposed or overlapping manner, the cutting edge 28c being protected from unintentional external engagement by the cutting blade 30b and the cutting edge 30c by the cutting blade 28b.

In the event of a disturbance occurring during the surgical operation, for example in the event of a disturbance due to a power failure or the like, the scissor element 30 cooperating with the shaft 26a is automatically axially displaced by the restoring force of the spring discs 62 arranged on the shaft 26a and the scissor blade 28b thus moved into the open position relative to the scissor blade 30b (Figure 11b). Removal of the two cutting blades 28b and 30b from the patient's eye is substantially prevented in the open position, without injuring or tearing the highly sensitive tissue.

If, in the assembled state according to Figure 19 or 20, the first bushing 32 applies an axially orientated force to the bushing 72 in the direction of the arrow Z' (Figure 19), the parts 32,72,82 and 69 can be axially displaced against the restoring force of the second compression spring 84. A displacement of the individual elements 32,72,82 and 69 of the mounting 64 arranged on the housing 48 allows the scissor elements 28 and 30 to be relatively displaced such that the two cutting blades 28b and 30b are moved into the so-called closed position in which they adopt a substantially overlapping position relative to one another (Figure 11a). In the overlapped position of the two cutting blades 28b, 30b, the surgeon can remove the cutting instrument 24 from the eye without injuring the tissue.

**Claims**

1. Electromechanical drive system for a device designed for carrying out intraocular surgical cutting functions, with two elongate scissor elements (28,30) which are arranged in a guide tube (34), are each provided with a cutting blade at the front end and of which at least the second scissor element (30) cooperates with an electric driving motor (26) and is linearly displaceable relative and parallel to the stationary first scissor element (28) between an open and a closed position of the cutting blades (28b,30b) arranged thereon for separating the intraocular tissue, characterised in that, for opening and closing the two cutting blades (28b,30b), at least the second scissor element (30) which is displaceable relative and linearly to the first scissor element (28) can be driven in a vibrating and simultaneously oscillating manner owing to additional inductive loading and in that a first amplitude value and a first frequency value is applied at the driving motor (26) for vibration and a second amplitude value and a second frequency value for oscillation, wherein, of the respectively predetermined values, the first amplitude value is smaller than the second amplitude value and the first frequency value greater than the second frequency value.

2. Electromechanical drive system according to claim 1, characterised in that the driving motor (26) can be activated by an electric circuit (95) and a controller (90) connected thereto selectively for the linear movement or for the vibrating or oscillating movement or for the simultaneous vibrating and oscillating movement of the moving scissor element (30) with respect to the stationary scissor element (28).

3. Electromechanical drive system according to claims 1 and 2, characterised in that the electric circuit (95) cooperating with the driving motor (26) comprises a potentiometer (94), several switches (96,106,110,102), several amplifiers (98,100,104), a first oscillator (105), a second oscillator (108) provided with a control element (112) and a multivibrator (116) which are coupled to one another for carrying out individual movement functions, the driving motor (26) being activatable by means of the individual elements depending on the selected movement function.

4. Electromechanical drive system according to claims 1 to 3, characterised in that the driving motor (26) can be excited with direct current for the oscillating movement of the second scissor element (30) and with superimposed alternating current for the simultaneous vibrating or oscillating movement.

5. Electromechanical drive system according to claim 1, characterised in that the predetermined first frequency value for the vibrating movement is greater than 200 Hz and the predetermined second frequency value for the oscillating movement is between 1 and 5 Hz.

6. Electromechanical drive system according to claims 1 and 2, characterised in that the second scissor element (30) is positioned with respect to the first scissor element (28) in any open position of the two scissor blades (30b,28b) relative to one another by a first movement function transmitted by the electric circuit (95) and the driving motor (26) and the second scissor element (30) can be driven simultaneously in a vibrating or oscillating manner with high frequency and low linear displacement in this position.

7. Electromechanical drive system according to claims 1 and 2, characterised in that the second scissor element (30) can be moved to and fro linearly with respect to the first scissor element (28) with a variably adjustable frequency between 1 Hz and 4 Hz by a second movement function produced by the electric circuit (95) via the driving motor (26) and can additionally be driven in a vibrating or oscillating manner at relatively high frequency.

8. Electromechanical drive system according to claims 1 and 2, characterised in that the reciprocating second scissor element (30) can also be driven in a vibrating or oscillating manner with respect to the first scissor element (28) by a first and a superimposed second signal by means of the third movement function produced by the electric circuit (95) via the driving motor (26).

9. Electromechanical drive system according to claims 1 and 2, characterised in that the second scissor element (30) is displaceable with respect to the first scissor element (28) owing to a supplied negative signal initially into an open position of the scissor blades (30b,28b) and can then be returned owing to a positive signal into a closed position with overlapped scissor blades (30b,28b) for removal from the tissue by means of a fourth movement function produced by the electric circuit (95) via the driving motor (26).

10. Device for carrying out intraocular surgical cutting functions with a electromechanical drive system according to claim 1, consisting of a housing (48) designed as a hand grip, an electric driving motor (26) arranged therein and a cutting instrument (24) which comprises a guide tube (34) which is designed as a hollow needle and is orientated in the axial direction of the housing (48) as well as two scissor elements (28,30) which are arranged in parallel therein, are provided with a respective cutting blade (28b,30b) at the front end projecting from the housing (48) and of which at least one scissor element cooperates with the driving motor (26) for a relative movement orientated with respect to the other scissor element, characterised in that the guide tube (34) with the two scissor elements (28,30) arranged therein are mounted coaxially in a headpiece (76) which is arranged in the same axial direction on the housing (48) and is displaceable relative thereto and in that the first scissor element (28) is stationarily connected to the headpiece (76) by one end and is provided with the first cutting blade (28b) at the other end and the second scissor element (30) cooperates with the shaft (26a) of the electric driving motor (26) by one end and has the second cutting blade (30b) at the other end, wherein at least the second scissor element (30) which is displaceable relative and linearly to the first scissor element (28) can be driven in a vibrating and simultaneously oscillating manner, and the two cutting blades (28b,30b) are arranged in an upwardly orientated manner on the scissor elements (28,30) with respect to the longitudinal axis (X') of the guide tube (34).

11. Device according to claim 10, characterised in that the first cutting blade (28b) of the stationary scissor element (28) is provided with a cutting edge (28c) which is inclined forward with respect to the front edge (34b) of the guide tube (34).

12. Device according to claim 11, characterised in that the cutting edge (28c) on the first cutting blade (28b) is inclined forward at an acute angle ($\alpha''$) to the longitudinal axis (X') of the guide tube (34).

13. Device according to claims 11 and 12, characterised in that the cutting edge (28c) of the first cutting blade (28b) is rectilinear or concave in design.

14. Device according to claim 10, characterised in that the second cutting blade (30b) of the moving scissor element (30) is provided with a convexly designed cutting edge (30c) on the side facing the cutting edge (28c) of the first cutting blade (28b).

15. Device according to claim 10, characterised in that the guide tube (34) with the two scissor elements (28,30) arranged therein is arranged in a first and second hollow cylindrical bushing (32,38) and these are mounted and fixed together coaxially in the headpiece (76) as a constructional unit and in that, owing to a movement of the headpiece (76) orientated in the direction of the housing (48) and acting against the restoring force of the first compression spring (78), the first bushing (32) fixed therein is released and the constructional unit designed as a cutting instrument (24) can then be removed from the headpiece (76).

16. Device according to claim 15, characterised in that the cutting instrument (24) comprising the two scissor elements (28,30), the bushings (32,38) and the guide tube (34) is held detachably in the headpiece (76) by several catch elements (74) which are arranged circumferentially in the headpiece (76) and can be brought into engagement with an annular groove (32f) of the first bushing (32).

17. Device according to claims 10 and 15, characterised in that the shaft (26a) of the electric driving motor (26) cooperates via a coupling element (60) which can be brought into engagement with the second bushing (38) for the respective movement of the second scissor element (30).

18. Device according to claim 11, characterised in that the first scissor element (28) can be displaced by means of the first bushing (32) cooperating therewith against the restoring force of a second compression spring (84) manually in the direction of the housing (48) relative to the second scissor element (30) cooperating with the electric driving motor (26) into an overlapped position of the two cutting blades (28b,30b) for removal from the tissue.

**Patentansprüche**

1. Elektromechanisches Antriebssystem für eine zur Durchführung augenchirurgischer Schneidfunktionen ausgebildete Vorrichtung, mit zwei in einem Führungsrohr (34) angeordneten und am vorderen Ende jeweils mit einem Schneidblatt versehenen länglichen Scherengliedern (28,30), von welchen mindestens das zweite Scherenglied (30) mit einem elektrischen Antriebsmotor (26) wirkverbunden und zum Trennen des intraokularen Gewebes relativ und parallel zu dem feststehenden ersten Scherenglied (28) zwischen einer offenen und einer geschlossenen Stellung der daran angeordneten Schneidblätter (28b,30b) linear verschiebbar ist, **dadurch gekennzeichnet**, dass zum Öffnen und Schliessen der beiden Schneidblätter (28b,30b) mindestens das relativ und linear zu dem ersten Scherenglied (28) verschiebbare zweite Scherenglied (30) infolge zusätzlicher induktiver Beaufschlagung vibrierend und simultan oszillierend antreibar ist, und dass an dem Antriebsmotor (26) für die Vibration eine erste Amplitudengrösse sowie eine erste Frequenzgrösse und für die Oszillation eine zweite Amplitudengrösse sowie eine zweite Frequenzgrösse anliegt, wobei von den jeweils vorbestimmten Grössen die erste Amplitudengrösse kleiner als die zweite Amplitudengrösse und die erste Frequenzgrösse grösser als die zweite Frequenzgrösse ist.

2. Elektromechanisches Antriebssystem nach Anspruch 1, **dadurch gekennzeichnet**, dass der Antriebsmotor (26) von einem elektrischen Stromkreis (95) sowie einer damit verbundenen Steuereinrichtung (90) wahlweise für die Linearbewegung oder für die Vibrations- oder Oszillationsbewegung beziehungsweise für die simultane Vibrations- und Oszillationsbewegung des beweglichen Scherenglieds (30) in bezug auf das feststehende Scherenglied (28) aktivierbar ist.

3. Elektromechanisches Antriebssystem nach den Ansprüchen 1 und 2, **dadurch gekennzeichnet**, dass der mit dem Antriebsmotor (26) wirkverbundene elektrische Stromkreis (95) zur Durchführung einzelner Bewegungsfunktionen gekoppelt miteinander verbunden einen Potentiometer (94), mehrere Schalter (96,106,110,102), mehrere Verstärker (98,100,104), einen ersten Oszillator (105), einen mit einem Steuerelement (112) versehenen zweiten Oszillator (108) sowie einen Multivibrator (116) umfasst, wobei mittels der einzelnen Elemente der Antriebsmotor (26) in Abhängigkeit der gewählten Bewegungsfunktion entsprechend aktivierbar ist.

**4.** Elektromechanisches Antriebssystem nach den Ansprüchen 1 bis 3, **dadurch gekennzeichnet**, dass der Antriebsmotor (26) für die Oszillationsbewegung des zweiten Scherengliedes (30) mit Gleichstrom und für die simultane Vibrations- oder Oszillationsbewegung mit überlagertem Wechselstrom erregbar ist.

**5.** Elektromechanisches Antriebssystem nach Anspruch 1**, dadurch gekennzeichnet**, dass die vorbestimmte erste Frequenzgrösse für die Vibrationsbewegung grösser als 200 Hz ist und die vorbestimmte zweite Frequenzgrösse für die Oszillationsbewegung zwischen 1 bis 5 Hz beträgt.

**6.** Elektromechanisches Antriebssystem nach den Ansprüchen 1 und 2, **dadurch gekennzeichnet**, dass durch eine von dem elektrischen Stromkreis (95) und dem Antriebsmotor (26) übertragene erste Bewegungsfunktion das zweite Scherenglied (30) in bezug auf das erste Scherenglied (28) in beliebig geöffneter Stellung der beiden Scherenblätter (30b,28b) zueinander positioniert wird und in dieser Stellung das zweite Scherenglied (30) gleichzeitig mit hoher Frequenz und geringer linearer Verschiebung vibrierend oder oszillierend antreibbar ist.

**7.** Elektromechanisches Antriebssystem nach den Ansprüchen 1 und 2, **dadurch gekennzeichnet**, dass durch eine von dem elektrischen Stromkreis (95) über den Antriebsmotor (26) bewirkte zweite Bewegungsfunktion das zweite Scherenglied (30) in bezug auf das erste Scherenglied (28) mit einer variabel einstellbaren Frequenz zwischen 1 Hz und 4 Hz linear hin- und herbewegbar und zusätzlich mit relativ hoher Frequenz vibrierend oder oszillierend antreibbar ist.

**8.** Elektromechanisches Antriebssystem nach den Ansprüchen 1 und 2, **dadurch gekennzeichnet**, dass durch eine von dem elektrischen Stromkreis (95) über den Antriebsmotor (26) bewirkte dritte Bewegungsfunktion das hin- und herbewegbare zweite Scherenglied (30) zusätzlich mit einem ersten und einem überlagerten zweiten Signal vibrierend oder oszillierend in bezug auf das erste Scherenglied (28) antreibbar ist.

**9.** Elektromechanisches Antriebssystem nach den Ansprüchen 1 und 2, **dadurch gekennzeichnet**, dass durch eine von dem elektrischen Stromkreis (95) über den Antriebsmotor (26) bewirkte vierte Bewegungsfunktion das zweite Scherenglied (30) in bezug auf das erste Scherenglied (28) infolge eines zugeführten Negativsignals zuerst in eine geöffnete Position der Scherenblätter (30b,28b) verschiebbar und anschliessend infolge eines Positivsignals in eine geschlossene Position mit überlappten Scherenblättern (30b,28b) zum Entfernen aus dem Gewebe zurückführbar ist.

**10.** Vorrichtung zur Durchführung augenchirurgischer Schneidfunktionen mit einem elektromechanischen Antriebssystem gemäss Anspruch 1, bestehend aus einem als Handgriff ausgebildeten Gehäuse (48), einem darin angeordneten elektrischen Antriebsmotor (26) und einem Schneidinstrument (24), welches ein als Hohlnadel ausgebildetes und in axialer Richtung des Gehäuses (48) orientiertes Führungsrohr (34) sowie zwei parallel zueinander darin angeordnete und am vorderen aus dem Gehäuse (48) herausragenden Ende jeweils mit einem Schneidblatt (28b,30b) versehene Scherenglieder (28,30) umfasst, von welchen mindestens das eine Scherenglied für eine in bezug auf das andere Scherenglied orientierte Relativbewegung mit dem Antriebsmotor (26) wirkverbunden ist, **dadurch gekennzeichnet**, dass das Führungsrohr (34) mit den beiden darin angeordneten Scherengliedern (28,30) koaxial in einem mit gleicher Achsrichtung am Gehäuse (48) angeordneten und relativ dazu verschiebbaren Kopfstück (76) gelagert sind, und dass das erste Scherenglied (28) mit dem einen Ende feststehend mit dem Kopfstück (76) verbunden und am anderen Ende mit dem ersten Schneidblatt (28b) versehen ist und das zweite Scherenglied (30) mit dem einen Ende mit dem Achskörper (26a) des elektrischen Antriebsmotors (26) wirkverbunden ist und an dem anderen Ende das zweite Schneidblatt (30b) aufweist, wobei mindestens das zweite Scherenglied (30) relativ sowie linear zu dem ersten Scherenglied (28) verschiebbar und simultan vibrierend sowie oszillierend bewegbar ist und die beiden Schneidblätter (28b,30b) in bezug auf die Längsachse (X') des Führungsrohres (34) nach oben orientiert an den Scherengliedern (28,30) angeordnet sind.

**11.** Vorrichtung nach Anspruch 10, **dadurch gekennzeichnet**, dass das erste Schneidblatt (28b) des feststehenden Scherengliedes (28) mit einer in bezug auf die Stirnkante (34b) des Führungsrohres (34) vorwärts geneigten Schneidkante (28c) versehen ist.

**12.** Vorrichtung nach Anspruch 11, **dadurch gekennzeichnet**, dass die Schneidkante (28c) an dem ersten Schneidblatt (28b) in bezug auf die Längsachse (X') des Führungsrohres (34) unter spitzem

Winkel ($\alpha''$) vorwärts geneigt angeordnet ist.

**13.** Vorrichtung nach den Ansprüchen 11 und 12, **dadurch gekennzeichnet**, dass die Schneidkante (28c) des ersten Schneidblattes (28b) geradlinig oder konkav ausgebildet ist.

**14.** Vorrichtung nach Anspruch 10, **dadurch gekennzeichnet**, dass das zweite Schneidblatt (30b) des beweglichen Scherengliedes (30) an der der Schneidkante (28c) des ersten Schneidblattes (28b) zugewandten Seite mit einer konvex ausgebildeten Schneidkante (30c) versehen ist.

**15.** Vorrichtung nach Anspruch 10, **dadurch gekennzeichnet**, dass das Führungsrohr (34) mit den beiden darin angeordneten Scherengliedern (28,30) in einem ersten und zweiten hohlzylindrischen Hülsenkörper (32,38) angeordnet und diese zusammen als eine Baueinheit koaxial in dem Kopfstück (76) gelagert und derart fixiert sind, und dass durch eine in Richtung des Gehäuses (48) orientierte und gegen die Rückstellkraft einer ersten Druckfeder (78) wirkende Bewegung des Kopfstücks (76) der darin fixierte erste Hülsenkörper (32) freigegeben und anschliessend die als Schneidinstrument (24) ausgebildete Baueinheit aus dem Kopfstück (76) herausziehbar sind.

**16.** Vorrichtung nach Anspruch 15, **dadurch gekennzeichnet**, dass das mit den beiden Scherengliedern (28,30), den Hülsenkörpern (32,38) und dem Führungsrohr (34) versehene Schneidinstrument (24) durch mehrere in Umfangsrichtung im Kopfstück (76) angeordnete und mit einer Ringnut (32f) des ersten Hülsenkörpers (32) in Eingriff bringbare Rastelemente (74) auswechselbar in dem Kopfstück (76) gehalten ist.

**17.** Vorrichtung nach den Ansprüchen 10 und 15, **dadurch gekennzeichnet**, dass der Achskörper (26a) des elektrischen Antriebsmotors (26) für die jeweilige Bewegung des zweiten Scherengliedes (30) über ein mit dem zweiten Hülsenkörper (38) in Eingriff bringbares Kupplungsglied (60) wirkverbunden ist.

**18.** Vorrichtung nach Anspruch 11, **dadurch gekennzeichnet**, dass das erste Scherenglied (28) mittels des damit wirkverbundenen ersten Hülsenkörpers (32) entgegen der Rückstellkraft einer zweiten Druckfeder (84) manuell in Richtung des Gehäuses (48) relativ zu dem mit dem elektrischen Antriebsmotor (26) wirkverbundenen zweiten Scherenglied (30) in eine überlappte Stellung der beiden Schneidblätter (28b,30b) zum Entfernen aus dem Gewebe verschiebbar ist.

**Revendications**

**1.** Système de commande électromécanique destiné à un dispositif conçu pour réaliser des fonctions d'incision en chirurgie oculaire, avec deux éléments de cisaillage longitudinaux (28,30) placés dans un tube de guidage (34) et munis respectivement à l'extrémité avant d'une lame d'incision, dont au moins le deuxième élément de cisaillage (30) est relié par action coordonnée à un moteur de commande électrique (26) et peut se déplacer linéairement, de façon relative et parallèle au premier élément de cisaillage fixe (28), entre une position ouverte et une position fermée des lames d'incision (28b,30b) placées dessus, afin de sectionner le tissu intraoculaire, **caractérisé en ce** que, afin d'ouvrir et de fermer les deux lames d'incision (28b,30b), au moins le deuxième élément de cisaillage (30), pouvant se déplacer de façon relative et linéaire par rapport au premier élément de cisaillage (28), peut être actionné de façon vibrante et oscillante en même temps, par suite d'une alimentation inductive supplémentaire, et qu'au moteur de commande (26) sont adjointes une première amplitude et une première fréquenoe pour la vibration, ainsi qu'une deuxième amplitude et une deuxième fréquence pour l'oscillation, ces grandeurs étant chacune prédéfinies et la première amplitude étant inférieure à la deuxième amplitude, tandis que la première fréquence est supérieure à la deuxième fréquence.

**2.** Système de commande électromécanique selon la revendication 1, **caractérisé en ce** que le moteur de commande (26) peut être actionné par un circuit électrique (95) ainsi que par un mécanisme de commande (90) qui lui est relié, au choix, pour le mouvement linéaire ou pour le mouvement vibratoire ou oscillatoire, ou bien pour le mouvement vibratoire et oscillatoire simultané de l'élément de cisaillage mobile (30) par rapport à l'élément de cisaillage fixe (28).

**3.** Système de commande électromécanique selon les revendications 1 et 2, **caractérisé en ce** que le circuit électrique (95), relié par action coordonnée au moteur de commande (26), comprend, afin de réaliser différentes fonctions de mouvement, un potentiomètre (94), plusieurs commutateurs (96,106,110,102), plusieurs amplificateurs (98,100,104), un premier oscillateur (105), un deuxième oscillateur (108) muni d'un élément de commande (112), ainsi qu'un multivibrateur (116), ces éléments étant couplés les uns aux autres, le moteur de commande (26) pouvant être actionné, à l'aide de ces différents éléments, en fonction du mouvement choisi.

**4.** Système de commande électromécanique selon les revendications 1 à 3 **caractérisé en ce** que le moteur de commande (26) peut être excité avec du courant continu pour le mouvement oscillatoire du deuxième élément de cisaillage (30) et avec du courant alternatif superposé pour le mouvement simultané vibratoire ou oscillatoire.

**5.** Système de commande électromécanique selon la revendication 1, **caractérisé en ce** que la première fréquence prédéfinie pour le mouvement vibratoire est supérieure à 200 Hz, tandis que la deuxième fréquence prédéfinie pour le mouvement oscillatoire se situe entre 1 et 5 Hz.

**6.** Système de commande électromécanique selon les revendications 1 et 2, **caractérisé en ce** que, par un premier mouvement transmis par le circuit électrique (95) et le moteur de commande (26), le deuxième élément de cisaillage (30) vient se placer, par rapport au premier élément de cisaillage (28), dans n'importe quelle position ouverte des deux lames d'incision (30b,28b) l'une par rapport à l'autre, et que, dans cette position, le deuxième élément de cisaillage (30) peut être actionné de façon vibrante ou oscillante, à la fois avec une fréquence élevée et un faible déplacement linéaire.

**7.** Système de commande électromécanique selon les revendications 1 et 2, **caractérisé en ce** que, par un deuxième mouvement causé par le circuit électrique (95) par le biais du moteur de commande (26), le deuxième élément de cisaillage (30) peut reculer et avancer de manière linéaire par rapport au premier élément de cisaillage (28), avec une fréquence réglable de façon variable entre 1 et 4 Hz, et peut en outre être actionné de façon vibrante ou oscillante avec une fréquence relativement élevée.

**8.** Système de commande électromécanique selon les revendications 1 et 2, **caractérisé en ce** que, par un troisième mouvement causé par le circuit électrique (95) par le biais du moteur de commande (26), le deuxième élément de cisaillage (30) pouvant avancer et reculer, peut en outre être actionné de façon vibrante ou oscillante, par rapport au premier élément de cisaillage (28), à l'aide d'un premier signal puis d'un deuxième signal superposé.

**9.** Système de commande électromécanique selon les revendications 1 et 2, **caractérisé en ce** que, par un quatrième mouvement causé par le circuit électrique (95) par le biais du moteur de commande (26), le deuxième élément de cisaillage (30) peut tout d'abord se déplacer par rapport au premier élément de cisaillage (28), par suite de la transmission d'un signal négatif, de sorte que les deux lames d'incision (30b,28b) se trouvent dans une position ouverte, puis, afin d'être retiré du tissu, qu'il peut être ramené, par suite d'un signal positif, de sorte que les lames d'incision (30b,28b) se trouvent dans une position fermée et se superposent.

**10.** Dispositif permettant de réaliser des fonctions d'incision en chirurgie oculaire, muni d'un système de commande électromécanique selon la revendication 1, composé d'un boîtier (48) conçu en forme de poignée, d'un moteur de commande électrique (26) placé dans le boîtier et d'un instrument d'incision (24), comprenant lui-même un tube de guidage (34) en forme de canule, orienté dans la direction axiale du boîtier (48), ainsi que deux éléments de cisaillage (28,30), placés dedans parallèlement l'un à l'autre et munis chacun, à leur extrémité avant dépassant du boîtier (48), d'une lame d'incision (28b,30b), au moins l'un de ces éléments de cisaillage étant relié par action coordonnée au moteur de commande (26) afin d'obtenir un mouvement relatif par rapport à l'autre élément de cisaillage, **caractérisé en ce** que le tube de guidage (34) avec les deux éléments de cisaillage (28,30) placés dans celui-ci, sont logés de façon coaxiale dans une pièce de tête (76), disposée sur le boîtier (48) dans la même direction axiale et de façon relativement mobile par rapport à celui-ci, et que le premier élément de cisaillage (28) est relié de façon fixe, à une extrémité, à la pièce de tête (76) et est muni, à l'autre extrémité, de la première lame d'incision (28b), tandis que le deuxième élément de cisaillage (30) est relié par action coordonnée, à une extrémité, au corps d'essieu (26a) du moteur de commande électrique (26), et est muni, à l'autre extrémité, de la deuxième lame d'incision (30b), au moins le deuxième élément de cisaillage (30) pouvant se déplacer de façon relative et linéaire par rapport au premier élément de cisaillage (28) et pouvant bouger de façon à la fois vibrante et oscillante, et les deux lames d'incision (28b,30b) étant placées sur les éléments de cisaillage (28,30) de manière orientée vers le haut par rapport à l'axe longitudinal (X') du tube de guidage (34).

**11.** Dispositif selon la revendication 10, **caractérisé en ce** que la première lame d'incision (28b) de l'élément de cisaillage fixe (28) est munie d'une arête de coupe (28c) inclinée vers l'avant par rapport au bord avant (34b) du tube de guidage (34).

**12.** Dispositif selon la revendication 11, **caractérisé en ce** que l'arête de coupe (28c) est placée sur la première lame d'incision (28b) de manière inclinée vers l'avant par rapport à l'axe longitudinal (X') du tube de guidage (34), selon un angle aigu ($\alpha''$).

**13.** Dispositif selon les revendications 11 et 12, **carac-**

**térisé en ce** que l'arête de coupe (28c) de la première lame d'incision (28b) présente une forme rectiligne ou concave.

**14.** Dispositif selon la revendication 10, **caractérisé en ce** que la deuxième lame d'incision (30b) de l'élément de cisaillage mobile (30), présente, sur son côté tourné vers l'arête de coupe (28c) de la première lame d'incision (28b), une arête de coupe (30c) de forme convexe.

**15.** Dispositif selon la revendication 10, **caractérisé en ce** que le tube de guidage (34), ainsi que les deux éléments de cisaillage (28,30) placés dans celui-ci, sont disposés dans un premier et un deuxième manchon en forme de cylindre creux (32,38) et que ces derniers sont logés et fixés ensemble de façon coaxiale, dans la pièce de tête (76), en tant qu'unité modulaire, de telle manière que le premier manchon (32) fixé dans cette unité est débloqué par un mouvement de la pièce de tête (76), orienté en direction du boîtier (48) et venant s'opposer à la force de rappel d'un premier ressort à pression (78), et que l'unité modulaire conçue en tant qu'instrument d'incision (24) peut ensuite être retirée de la pièce de tête (76).

**16.** Dispositif selon la revendication 15, **caractérisé en ce** que l'instrument d'incision (24), muni des deux éléments de cisaillage (28,30), des manchons (32,38) et du tube de guidage (34), est maintenu dans la pièce de tête (76) à l'aide de plusieurs éléments encliquetables (74) disposés dans la pièce de tête (76) sur la périphérie et pouvant s'enclencher avec la rainure annulaire (32f) du premier manchon (32).

**17.** Dispositif selon les revendications 10 et 15, **caractérisé en ce** que le corps d'essieu (26a) du moteur de commande électrique (26) est relié par action coordonnée par le biais d'un élément de couplage (60) pouvant être engrené avec le deuxième manchon (38), pour chaque mouvement du deuxième élément de cisaillage (30).

**18.** Dispositif selon la revendication 11, **caractérisé en ce** que le premier élément de cisaillage (28), afin d'être retiré du tissu, peut être déplacé manuellement, en direction du boîtier (48) et de façon relative par rapport au deuxième élément de cisaillage (30) relié par action coordonnée au moteur de commande électrique (26), au moyen du premier manchon (32) qui lui est relié par action coordonnée, en s'opposant à la force de rappel d'un deuxième ressort à pression (84), de sorte que les deux lames d'incision (28b, 30b) se trouvent superposées.

FIG. I

EP 0 610 247 B1

FIG. 2

FIG. 3

FIG. 4

FIG. 5

FIG. 6

FIG. 7

FIG. 8

FIG. 9

FIG. 10

FIG.11c

FIG.11b

FIG.11a

FIG.12

FIG.13

MONOSTABLE MULTIVIBRATOR

POWER AMP

MOTOR

AMP

OSCILLATOR 500 HZ

OSCILLATOR 1-4 HZ

AMP

+V$_{CC}$

−V$_{CC}$

LINEAR MULTIPLE CUT
OSCILLATORY
LINEAR

MULTIPLE POWER
OSCILLATORY

MODE

HANDPIECE

CUTRATE

FIG.16

FIG.17

FIG.14

FIG.15

FIG.18

FIG. 19a

FIG. 19

FIG. 20a

FIG. 20

EP 0 610 247 B1